# FASCICULE DE BREVET EUROPEEN

(11) **EP 2 749 307 B1**
(45) Date de publication et mention de la délivrance du brevet: **11.03.2015**
(21) Numéro de dépôt: 13196537.8
(22) Date de dépôt: 10.12.2013
(51) Int. Cl.: A61M 5/32, A61B 17/34

(54) **Instrument d'intervention sous-cutanée et mandrin**
Instrument für subkutane Eingriffe, und Bohrvorrichtung
Subcutaneous intervention instrument and mandrel

(30) Priorité: 28.12.2012 FR 1262904
(43) Date de publication de la demande: 02.07.2014
(73) Titulaire: Haddad, Guy, 75008 Paris (FR)
(72) Inventeur: Haddad, Guy, 75008 Paris (FR)
(74) Mandataire: Novagraaf Technologies

(56) Documents cités:
- WO-A1-90/01349
- WO-A1-2005/117687
- WO-A2-2004/030731
- GB-A- 2 272 895
- US-A1- 2007 249 992

## Description

L'invention concerne un instrument d'intervention dermique ou sous-cutanée, en particulier derme ou hypoderme.

Le document US2007/0249992 Al, sur lequel le préambule de la revendication 1 est basé, montre un instrument d'intervention sous-cutanée comprenant un mandrin et une canule pourvue d'un tube d'injection et comprenant une spatule terminale qui prolonge le tube à son extrémité distale. Les documents WO 90/01349 Al et GB 2 272 895 A montrent des canules comprenant une extrémité distale atraumatique. Ces canules ne sont pas destinées à des opérations sous-cutanées mais pour passer à travers un joint sans l'abîmer. Ces canules ne sont donc pas destinées à être utilisées avec des mandrins.

WO 2005/117687 Al montre un mandrin pour intervention sous-cutanée, le mandrin présentant une tige pourvue d'une extrémité d'accès corporel, agencée de sorte à pouvoir s'engager de façon amovible dans une embase de raccordement creuse.

Parmi les problèmes que l'on souhaite résoudre, on notera :
- le traumatisme de certaines interventions ; risques de léser les tissus,
- le caractère non-indolore de certaines interventions, typiquement après intervention,
- la persistance de marques sur la peau, après interventions (cicatrices en particulier)
- la difficulté à agir efficacement dans ces tissus, tout en pouvant y effectuer des injections de produits (en particulier ceux utilisés en chirurgie esthétique, tel l'acide hyaluronique) et/ou y réaliser des prélèvements, avec le même instrument.

Afin d'apporter une solution à une partie au moins de ces problèmes, il est proposé l'instrument d'intervention selon la revendication 1.

On doit comprendre les adjectifs suivants comme suit :
- dermique ou sous-cutanée : comme relatif à la peau dans toute son épaisseur ; épiderme, derme et hypoderme. Ainsi, la canule pourra pénétrer jusqu'à l'hypoderme (là où sont les tissus graisseux sous-cutanés) et les injections de produit, via le tube précité, pourront se faire dans le derme,
- douce : comme atraumatique, par exemple arrondi,
- diamètre : comme relatif à une section, quand bien même il ne s'agirait pas strictement d'une section circulaire, mais par exemple ovale,
- connexion « Luer lock » : comme relatif à un embout de connexion rapide et fiable avec un tube, selon le mode le plus répandu de connexion actuellement exploité et commercialisé.

Une problématique complémentaire concerne :
- la facilité à réaliser, en intervention, un geste ayant un effet lissant du produit, la spatule passant après le produit pour l'aplanir (effet couteau à tartiner), évitant ainsi les flaques et les irrégularités.
- et/ou la possibilité d'atteindre le plan de clivage naturel (espace sous-cutané qui se décolle aisément grâce à la canule), et d'y demeurer, en intervention, ce qui favorisera l'uniformité d'une répartition de produit (lors d'une injection) ou d'un prélèvement de tissus, adipeux en particulier.

A cette fin, il est conseillé que la spatule :
- soit plate,
- et/ou s'étende dans le prolongement axial du tube.

En particulier pour les caractères atraumatiques et indolores, on recommande par ailleurs:
- que le tube présente un diamètre extérieur maximal et, transversalement à l'axe longitudinal, que la spatule présente une largeur comprise entre 0,6 et 1,3 fois le diamètre extérieur maximal,
- et/ou que le mandrin présente, pour ménager ledit conduit d'accès, un diamètre extérieur maximal prédéterminé, et le tube présente un diamètre extérieur maximal supérieur à 1,3 fois celui du mandrin,
- et même plus précisément et de préférence :
   - que le tube présente un diamètre extérieur constant, et, transversalement à l'axe longitudinal, que la spatule présente une largeur égale audit diamètre extérieur,
   - et/ou que le mandrin présente un diamètre extérieur constant (sauf à sa partie pointue), et le tube un diamètre extérieur constant égal (à 5% près) de celui du mandrin.

Dans les deux premiers cas, on peut jouer sur l'élasticité de la peau à l'endroit de l'accès percutané pour « absorber » les différences de diamètres, l'objectif étant d'éviter les cicatrices et marques disgracieuses qui peuvent en outre être douloureuses.

Encore pour ces aspects d'interventions atraumatiques et indolores, il est conseillé que le mandrin présente un axe longitudinal et une tige suivant cet axe, laquelle :
- est effilée à ou en direction de l'extrémité d'accès corporel, suivant ledit axe longitudinal, et/ou
- présente une partie tubulaire rectiligne terminée d'un côté par l'extrémité d'accès corporel qui est conique suivant ledit axe longitudinal.

Pour les mêmes buts visés, tout en favorisant les gestes du praticien (atteinte de l'hypoderme, manoeuvrabilité aisée du mandrin), on recommande :
- que, suivant l'axe longitudinal, l'embase creuse de la canule présente une longueur supérieure à la longueur du mandrin suivant cet axe,
- voire que la longueur du mandrin suivant cet axe soit comprises entre 1 et 3 cm et de préférence entre 1,5 et 2,5 cm.

Encore pour facilité et sécuriser les gestes d'intervention du praticien en favorisant l'asepsie, on conseille que :
- l'embase creuse et le mandrin soient adaptés pour que le mandrin puisse s'engager de façon amovible dans l'embase creuse,
- et/ou que l'embase creuse se termine par une partie évasée vers l'extérieur qui s'étend coaxialement au tube et reçoit le mandrin,
- et encore :
- que l'embase creuse de la canule définisse extérieurement une première poignée de prise en main,
- qu'à l'opposé de l'extrémité d'accès corporel, le mandrin soit fixé à une seconde poignée de prise en main, et
   - les première et seconde poignées sont chacune à facettes, et/ou
   - les première et seconde poignées présentent respectivement des premiers et seconds moyens de détrompage angulaire qui, engagés ensemble, bloquent angulairement et de façon réversible les première et seconde poignées l'une par rapport à l'autre et permettent de savoir dans quelle position angulaire est la spatule, et
   - la seconde poignée et/ou l'embase est pourvue d'un marqueur angulaire, sur une zone visible de dessus quand le tube d'injection ou de prélèvement est angulairement orienté pour que son orifice distal s'ouvre à un niveau inférieur à celui de la spatule.

A nouveau pour faciliter les gestes du praticien et les conditions d'asepsie, il est conseillé qu'à l'opposé de l'extrémité d'accès corporel, le mandrin est fixé à une poignée de prise en main terminée, à l'opposé du mandrin, par une surface plane d'appui permettant de poser la poignée de façon stable avec le mandrin dressé.

Dressé, le mandrin ne risquera pas d'être souillé par la surface sur laquelle il aura pu être posé.

Pour favoriser les gestes dans le plan de clivage naturel, et les injections et/ou les prélèvements lors de ces gestes, on conseille que l'orifice distal du tube soit situé près de l'extrémité distale de ce tube, dans la paroi latérale dudit tube.

Pour faciliter l'utilisation de l'instrument dans le milieu médical courant, via une solution simple et fiable, il est proposé que la canule :
- soit en outre pourvue d'une embase de raccordement proximal creuse présentant une connexion « Luer lock » et à laquelle le tube se raccorde à l'extrémité proximale, et
- l'embase creuse et le mandrin sont adaptés pour que le mandrin puisse s'engager de façon amovible dans l'embase creuse.

Est aussi visé le mandrin pour l'instrument médical. Le concernant, les problèmes que l'on souhaite résoudre sont en particulier liés à la réalisation d'une voie d'abord atraumatique, si possible indolore après intervention et qui favorise l'introduction transcutanée de la/d'une canule, par cette voie, y compris si nécessaire jusqu'en zone hypodermique.

Une solution proposée est que le mandrin présente une tige pourvue d'une extrémité d'accès corporel, pour ménager ledit conduit d'accès vers la zone sous-cutanée à atteindre et pour s'engager de manière amovible dans une embase creuse, la tige ayant une longueur inférieure à celle de l'embase. La longueur de la tige est axialement comprise entre 0,8 et 3 cm.

En particulier, la tige présente, à l'opposé de l'extrémité d'accès corporel, une poignée de prise en main ayant une plus grande section que celle de la tige.

D'autres caractéristiques et avantages de l'invention apparaîtront encore dans la description qui suit faite en référence aux dessins annexés donnés à titre d'exemple de réalisation préféré et dans lesquels:
- les figures 1,2 montrent en vue de dessus, successivement, un mandrin de percement de la peau et une canule d'intervention sous-cutanée dans l'extrémité proximale de laquelle est engagée le mandrin amovible,
- la figure 3 montrent en vue de côté la canule seule,
- la figure 4 schématise un embout de tube,
- la figure 5 schématise la coupe d'un derme, avec la canule engagée,
- les figures 12,13,14,15 montrent des variantes,

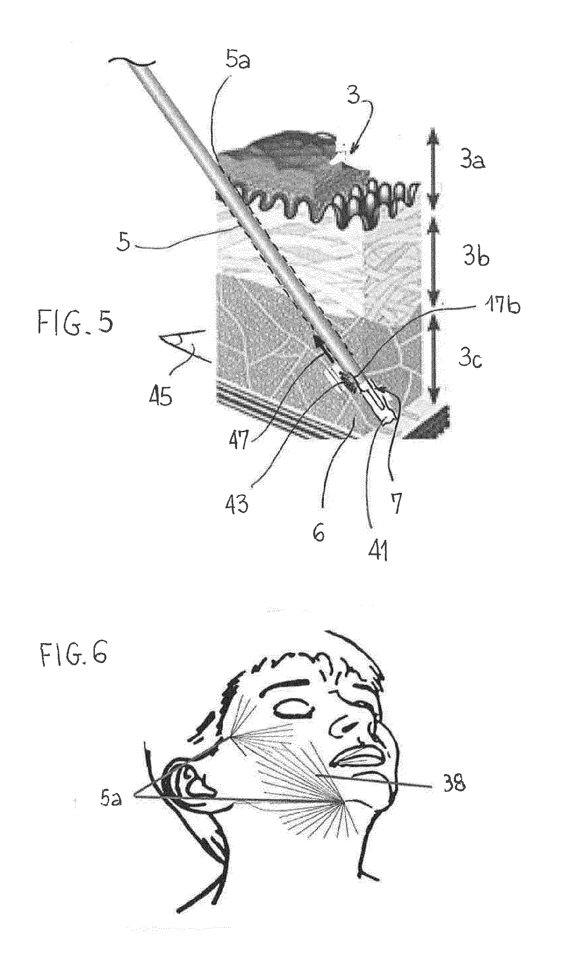

- et les figures 6,7,8,9,10,11 schématisent la technique d'intervention utilisant l'instrument précédemment présenté, dans l'une ou l'autre de ses variantes (solutions non limitatives).

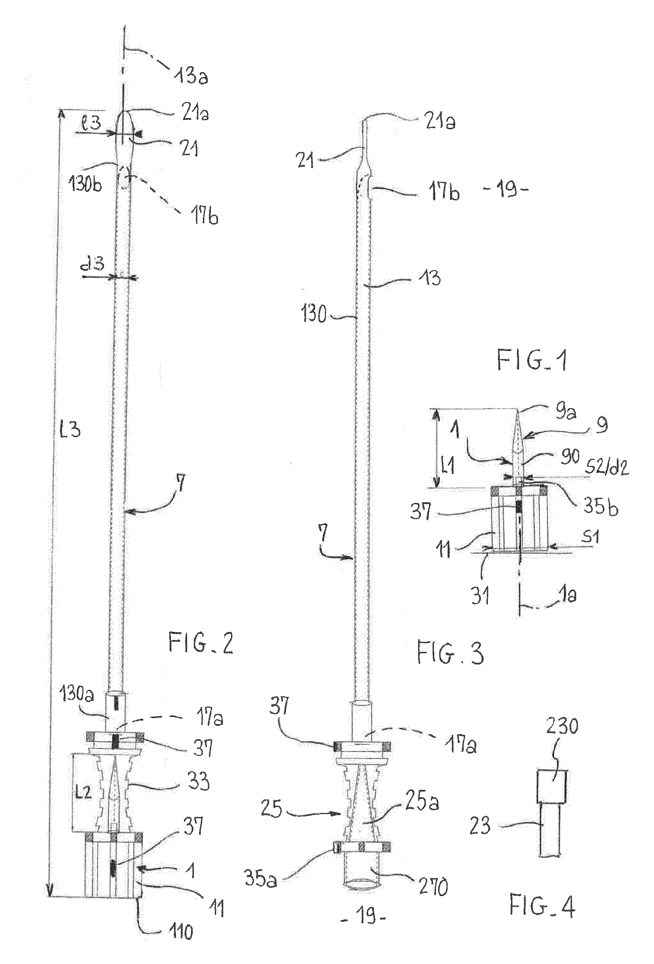

Figure 1 en particulier, on voit un mandrin 1, c'est-à-dire un poinçon visant à percer la peau 3, a priori jusqu'à l'hypoderme 3c (voir figure 5).

Le mandrin 1 forme (ou ici présente), suivant son axe longitudinal 1a, une tige 9 pourvue d'une extrémité d'accès corporel 9a permettant de ménager un conduit d'accès 5 transcutané.

A l'opposé axial, le mandrin présentera de préférence une poignée 11 de prise en main ayant une plus grande section S1 que celle S2 de la tige, ladite tige ayant axialement une longueur L1 entre 0,8cm et 3 cm. Ainsi, on favorisera la réalisation d'une voie d'accès non traumatique et ne devant pas créer de marques persistances sur la peau, après intervention.

L'action du mandrin 1 vise à permettre de créer le conduit d'accès 5 vers la zone sous-cutanée 6 à atteindre, une canule médicale 7 étant ensuite introduite jusqu'à cette zone sous-cutanée 6, par la voie d'accès 5.

Le mandrin 1 et la canule 7 forment ici l'instrument d'intervention sous-cutanée 10.

La canule est notablement plus longue axialement que le mandrin, comme montré notamment figures 2, 12-15.

Elle comprend :
- un tube 13 d'injection ou de prélèvement s'étendant suivant un axe longitudinal 13a et terminé, vers des extrémités respectivement proximale 130a et distale 130b, par un orifice 17a ou 17b communiquant avec l'extérieur 19,
- et une spatule 21 terminale présentant une extrémité atraumatique 21a à engager dans le conduit d'accès 5 et qui prolonge le tube 13 à son extrémité distale 130b.

Pour favoriser le geste dans l'hypoderme, on recommande que la spatule 21 s'étende dans le prolongement axial du tube.

Et pour un lissage favorable du produit qui pourra être apporté par le tube 13, typiquement de l'acide hyaluronique, avec un effet proche de celui d'un couteau à tartiner, visant à éviter ainsi les flaques et les irrégularités par aplanissement du produit apporté, on conseille que la spatule 21 soit plate.

Afin d'ailleurs notamment d'apporter le produit jusqu'à cette spatule en prévenant déjà les risques d'excès local, tout en favorisant la répartition à venir lorsque le praticien va lisser la zone en déplaçant la canule, il, est préféré que l'orifice 17b du tube 13 soit situé près de (ou à) l'extrémité distale 130b, dans la paroi latérale 130 dudit tube ; voir notamment figure 3.

Pour limiter notablement les risques de déchirure de la peau lors de l'introduction ou de la manoeuvre de la canule dans/sous la peau, puis de séquelles cicatricielles, il est prévu :
- que le tube 13 présente un diamètre extérieur maximal d3 (ce diamètre pouvant se réduire localement, notamment vers l'extrémité distale, pour réduire l'espace cutané occupé),
- et que, transversalement à l'axe longitudinal 13a, la spatule 21 présente une largeur (maximale) 13 comprise entre 0,6 et 1,3 fois ce diamètre extérieur maximal d3.

Pour réduire les risques au maximum, il est même recommandé que le tube présente un diamètre extérieur constant d3, et, que transversalement à l'axe longitudinal, la largeur maximale 13 de la spatule soit égale audit diamètre extérieur (à 5% près).

Dans un but similaire, on recommande que le mandrin 1 présente, pour ménager le conduit d'accès 5, un diamètre extérieur maximal prédéterminé d2 (correspondant à la section S2), le diamètre extérieur maximal d3 du tube étant inférieur à 1,3 fois celui d2 du mandrin (entre 0,6 et 1,3 fois a priori), la solution préférée étant à nouveau des diamètres extérieurs égaux (à 5% près ; d3=13=d2=S2).

Une raideur en flexion donnant une certaine souplesse au tube permettra au tube 13 d'être facile à guider sous le derme de façon précise, sans traumatisme.

Pour injecter ou prélever via le tube 13, depuis ou vers le tuyau médical 23 (figure 4), il est préféré:
- que la canule 7 soit pourvue d'une embase creuse 25 de raccordement proximal présentant une connexion « Luer lock » 270 et auquel le tube 13 se raccorde à l'extrémité proximale 130a, et
- que l'embase creuse 25 et le mandrin 1 soient adaptés pour que le mandrin puisse s'engager de façon amovible dans l'embase creuse (voir figures 2 et 12-15).

Pour son utilisation fonctionnelle, le tuyau médical 23 présentera lui-même une connexion « Luer lock » 230 compatible avec celle 270.

Quant à la longueur L2 suivant l'axe longitudinal 13a de cette embase creuse (hors la connexion 270), elle sera favorablement supérieure à la longueur axiale L1 du mandrin, ou du moins de sa tige pointue 9. Ainsi, on favorisera une bonne protection du mandrin, dans l'embout, sans faire passer la tige dans le tube 13, évitant ainsi que les diamètres soient compatibles et surtout que le diamètre du conduit d'accès 5 soit (notablement) inférieur au diamètre extérieur d3 du tube 13, avec le risque de traumatisme (marques, voire cicatrice) associé.

Pour favoriser cet engagement amovible et une manoeuvre pratique de l'instrument complet (mandrin et canule), il sera préféré que le mandrin 1 soit effilé, en direction de son extrémité d'accès corporel 9a, suivant l'axe longitudinal la de sa tige.

Plus précisément, la pointe de cette tige 9 sera, suivant cet axe (pour un centrage favorable dans l'embout, lui-même conique suivant l'axe 13a (ou en double cône inversé) :
- effilée à ou en direction de l'extrémité d'accès corporel 9a, ou
- pour assurer la réalisation d'un diamètre d'accès corporel 5 suffisant et un bon centrage dans l'embout, favorablement pourvu d'une partie tubulaire rectiligne 90 terminée, du côté distal, par une extrémité d'accès corporel 9a conique, centré sur l'axe 1a.

Concernant l'embase creuse 25, on recommande qu'alors elle se termine par un logement 25a intérieur évasé vers l'extérieur qui s'étendra coaxialement au tube 13 et recevra alors le mandrin (sa tige 9) ; voir figures 2 et 12-15.

Pour favoriser l'asepsie et la facilité d'utilisation du mandrin, on recommande par ailleurs qu'à l'opposé de l'extrémité d'accès corporel 9a, le mandrin 1 soit fixé à une poignée 11 de prise en main terminée, à l'opposé du mandrin, par une surface plane 110 d'appui permettant de poser la poignée de façon stable avec le mandrin dressé (voir figure 1 avec sa surface horizontale plane 31).

Présenté d'une autre manière, on préfèrera :
- que l'embase creuse 25 de la canule définisse extérieurement une première poignée 33 de prise en main,
- qu'à l'opposé de l'extrémité d'accès corporel 9a, le mandrin soit donc fixé à une seconde poignée 11 de prise en main, et
   - que les première et seconde poignées soient chacune à facettes, comme illustré figures 2,3 et 12-15, et/ou
   - que ces première et seconde poignées présentent respectivement des premiers et seconds moyens 35a,35b de détrompage angulaire (tels respectivement qu'une encoche recevant une courte patte) qui, engagés ensemble, bloquent angulairement et de façon réversible les première et seconde poignées l'une par rapport à l'autre et permettent de savoir dans quelle position angulaire est la spatule, et
   - que la seconde poignée 11 et/ou l'embase 25 soit pourvue d'un marqueur angulaire 37, sur une zone qui sera visible de dessus quand le tube d'injection ou de prélèvement 13 est angulairement orienté pour que son orifice distal 17b s'ouvre à un niveau inférieur à celui de la spatule 21 ; voir figures 2,3.

Ainsi, le praticien pourra, sans sortir la canule de la zone sous-cutanée 6, et après l'avoir engagée jusque vers le bout de sa zone d'intervention (voir plus loin), agir sur la canule par une action rétro-traçante (on prélève, ou on injecte le produit en retirant progressivement la canule du bout vers l'orifice d'entrée corporel 5a). On s'assurera ainsi d'un effet lissant si un produit a été apporté via le tube 13.

Le marqueur 37 pourra être un trait sur la poignée ou l'embase.

On doit ici comprendre poignée et embase au sens large, donc couvrant les pièces en elle-même et leur environnement, tel la partie du tube 13 proche de l'extrémité 130a.

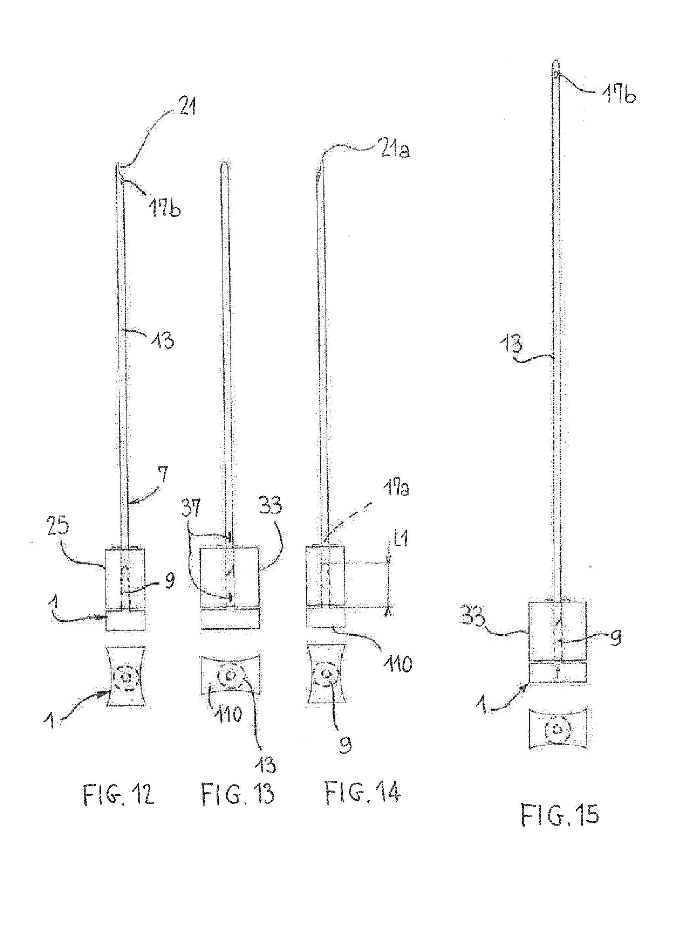

Figures 12 à 15, on a représenté deux variantes de la solution précédentes, respectivement figures 12-14 et figure 15. Leurs différences :
- tubes 13 allongés et de diamètres pouvant variés,
- extrémité de tige 9 de mandrin biseautée.

Pour résumer ce qui précède, on recommande un instrument 10 ayant les caractéristiques suivantes :

Pour le tube 13 de la canule 7, à son extrémité distale 130b :
- spatule 21 à « bout mousse », atraumatique,
- forme de spatule arrondie
- orifice latéral 17b situé en dessous du niveau de la spatule, en position opérationnelle de lissage de celle-ci (voir figure 5).

Pour le diamètre du tube 13 :
- gamme très large, en unité GAUGE, de 27G (le plus fin), à 12G (le plus gros).

Pour la longueur utile :
- longueur L3 de la canule, de l'extrémité distale de la spatule à la base 110 de la poignée 11 : de 4 cm à 18 cm

Pour la tige pointue 9 du mandrin 1 :
- bout biseauté,
- tige pleine,
- même diamètre que le tube 13 de la canule (à environ 5% près) : possible grâce à l'intrusion de la tige de ce mandrin uniquement dans l'embase creuse 25, et à sa longueur utile de tige d'environ 1.5 cm, de préférence.

Pour le mandrin 1 :
- repérage sur l'embase creuse 25 : marquage 37 opposé à l'orifice distal 17b.

Pour la résistance et flexibilité du tube 13 de la canule :
- constante grâce au rapport diamètre / longueur,
- équilibre entre flexibilité et résistance,
- matériau : acier inox.

Concernant l'utilisation de l'instrument 10, on notera maintenant ce qui suit : l'objectif est de créer des volumes, de réaliser des comblements et/ou de repositionner la graisse, typiquement dans les tissus graisseux sous-cutanés.

### 1. Concernant la sculpture des volumes :

Importance des tracés avant l'acte d'apport de produit de comblement prévu (tel l'acide hyaluronique). Ceux-ci permettent de définir, par des tracés 38 sur la peau, les zones de tissus à traiter et la quantité de produit nécessaire (nombre de seringues) pour chaque zone : véritable "maquette préparatoire" ; voir figure 6.

Avant de ménager le/chaque conduit d'accès 5 transcutané, on recommande une anesthésie des points d'entrée, tel ceux 5a figure 6, par exemple par injection de xylocaïne adrénalinée (0,5 ml en moyenne). Le produit se diffuse de façon a priori concentrique, anesthésiant la peau sur une surface plus ou moins circulaire de quelques millimètres de diamètre, surface suffisante pour l'introduction du mandrin 1.

Il est conseillé de bien définir tous les points d'entrée, qui sont réalisés de préférence dans le même temps (après l'anesthésie locale) ; Ce geste permet d'éviter les fautes d'asepsie.

La tige 9 du mandrin 1 traverse uniquement la couche cutanée, et on conseille de la (re)placer dans l'embase 25 immédiatement après avoir pratiqué la/les perforations.

Si une injection ou un prélèvement corporel (typiquement graisseux) est à effectuer via le tube 13, on conseille donc de ne retirer définitivement le mandrin 1 que quand tous les points d'entrée sont réalisés.

Il est alors de préférence posé debout, par sa face 110, tige 9 dressée.

On peut alors introduire le tube 13 adéquat de la canule dans le conduit d'abord 5 réalisé par le mandrin.

Il y a d'autant moins de risque de déchirure de la peau, et de séquelles cicatricielles, que les diamètres de la tige 9 du mandrin et du tube 13 de la canule ont des diamètres proches.

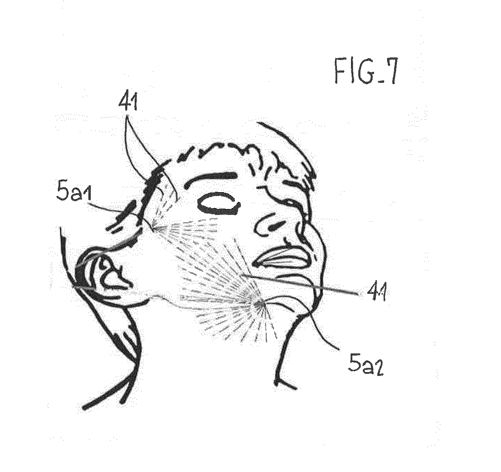

Mandrin retiré de l'embase 25, on peut adapter sur la connexion 270, une seringue d'anesthésique local dilué. Outre son rôle anesthésiant, ce liquide facilite la création de travées sous-cutanées 41 ; figures 5,7 (hydro-dissection).

De préférence, ces travées 41 correspondront, sous l'épiderme 3a et a priori également le derme 3b (figure 5), aux tracés 38 de surface, préparatoire, qui permettront de guider le geste du praticien.

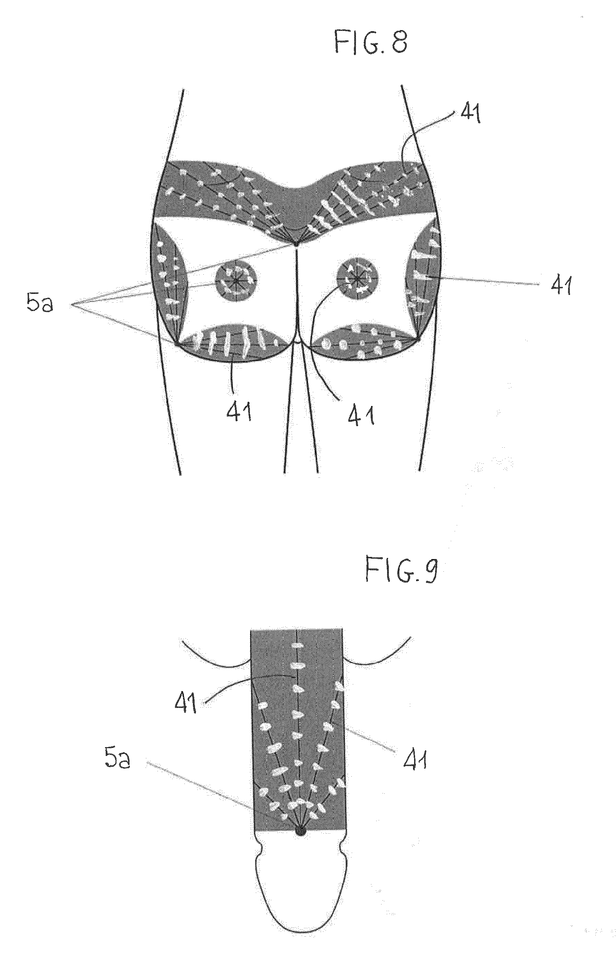

Le tube 13 de la canule est alors introduit, via les points 5a, a priori jusqu'à dans l'hypoderme 3c. Selon les parties du corps à traiter, la peau sera plus ou moins fine, et la surface plus ou moins grande. Il faut donc adapter le diamètre et la longueur du tube 13 aux besoins. De même, le plan d'injection 45 sera plus ou moins profond (figure 5): pour les fesses (figure 8), on recommande que le plan soit au contact de l'aponévrose des muscles fessiers et le tube 13 est a priori plus long et plus gros. Pour la pénoplastie (figure 9), la peau est généralement fine. L'injection est plus superficielle (typiquement sous le fascia), et le tube 13 est a priori plus fin.

Réalisation de travées 41 sous-cutanés (figure 7): véritables tunnels sous la peau, réalisés donc de préférence sous les tracés 38 ; geste totalement atraumatique, consistant en mouvements oscillants et de va-et-vient, sensiblement transversalement à la direction de la travée d'accès qui prolonge le conduit d'accès 5. On crée ainsi un plan uniforme (plan de clivage naturel) pour une bonne répartition du produit de comblement à injecter. En même temps est injecté le produit anesthésiant dilué.

La canule est tenue entre le pouce et l'index, par sa poignée 33, ce qui permet de contrôler son trajet. Le décollement sous la peau ne doit pas rencontrer une grande résistance. Si la (tige de la) canule bute, c'est qu'elle n'est pas dans le bon plan (plan de clivage naturel). Dans ce cas, on recommande de reculer, sans sortir la (tige de la) canule. Il est fortement conseillé de rester toujours dans le même plan. Il n'y a alors pas de risque notable de léser les tissus, la spatule étant à extrémité atraumatique.

On crée ainsi une sorte de logement, comparable à celui destiné à une prothèse mammaire.

Une fois les travées 41 finies, commence l'injection du produit de comblement.

Cette injection se fait en rétro-traçant :
- on injecte le produit (43, figure 5) alors que le tube est vers le fond des travées 41,
- et en retire progressivement la (tige de la) canule, en direction du trou d'accès 5a ; flèche 47 figure 5.

Le geste permet d'avoir un effet lissant du produit de comblement apporté, la spatule 21 étant située après (plus profondément, ou plus au fond de la travée, que) l'orifice distal 17b ; elle passe donc après le produit pour l'aplanir (effet couteau à tartiner), évitant ainsi les flaques et les irrégularités.

On recommande de ne pas revenir sur la zone où l'injection du produit de comblement à eu lieu : risque, sinon, de déplacer le produit et perdre l'effet lissant de la spatule.

Compte tenu de la maquette préparatoire, la quantité de produit a normalement pu être prédéfinie par zone, la répartition se faisant alors selon le nombre total de seringues de produit de comblement nécessaires (de 1 à 30, typiquement).

Généralement, on utilise pour un même acte un seul type de (tige de) canule (visage, pénoplastie), parfois deux (pectoraux, mollets) et même jusqu'à trois (fesses).

Les types d'intervention nécessitant une technique affirmées sont typiquement les suivantes :
- VISAGE : on recommande deux points d'entrée maximum de chaque côté du visage (pour un côté : points 5a1,5a2, figure 7, ici respectivement sur l'arcade zygomatique et en zone para-mentonnière).

Ces seuls points d'entrée permettent de traiter un visage dans son ensemble, et dans la majorité des cas une seule canule suffit (telle une tige de 18G ou 20G). Si les lèvres nécessitent une augmentation de volume, une (tige de) canule plus petite est utilisée (telle 27G).

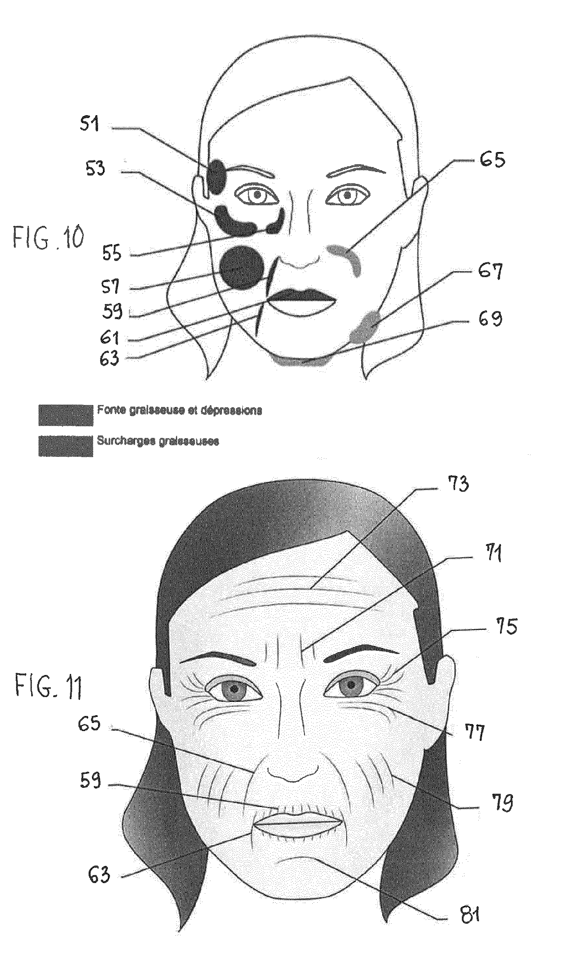

Toutes les augmentations typique de volume du visage peuvent être traitées par cette technique et par ces voies d'abord: tempes, pommettes, joues, vallée des larmes (creux sous l'oeil et sur la pommette)... ; voir le lipo-lissage de la figure 10.
- FESSES : de un à six points d'entrée, typiquement, a priori sans aucune cicatrice. On peut agir directement au niveau des zones inaccessibles par la ligne médiane (pli inter-fessier), et ce donc sans aucune cicatrice ni traumatisme des tissus. On utilise généralement des (tiges de) canules adaptées, de diamètres et de longueurs différents ; voir figure 8.

Cette technique permet de recréer la cambrure, de galber les parties centrales des fesses, de remplir les creux latéraux et de lisser les plis fessiers.

PECTORAUX: un à deux points d'entrée, typiquement, de chaque côté (latéraux ou inférieurs),

PENOPLASTIE: un seul point d'entrée, typiquement. La canule permet, de façon indolore et atraumatique, de retrouver le plan de clivage naturel (espace sous-cutané qui se décolle aisément grâce à la (tige de la) canule), ce qui va donner une répartition uniforme du produit. Une seule canule est a priori nécessaire (18G, 12cm) pour réaliser l'augmentation de volume et l'allongement voir figure 9.

### 2. Lipolissage :

Les volumes du visage se modifient avec l'âge ; voir figure 10. Les zones concernées sont typiquement : les tempes 51, les pommettes 53, les vallées des larmes 55, les creux de la joue 57, les sillon nasogéniens 59, les lèvres 61, les plis de l'amertume 63, les plis nasogéniens 65, les bajoues 67, le double menton (69).

Les premières étapes de ce type d'interventions sont communes avec ce qui précède : tracés, définition des zones à traiter (maquette préparatoire), nombre de seringues de produit de comblement prévues, et leur répartition, les zones de graisseuse surcharge étant bien circonscrites. Ensuite, points d'entrée, sous anesthésie locale, puis réalisation du plan sous cutané, dans le tissus graisseux sous cutané (sous l'hypoderme).

Phase particulière, propre à cette variante qu'est le lipo-lissage: désolidarisation de la graisse et son repositionnement ascensionnel.

Les cellules graisseuses sont séparées de leurs attaches par des mouvements de va-et-vient et en éventail.

La tige de la canule progresse dans la graisse sans difficultés. Une résistance signifiera a priori que la tige 13 n'est pas dans le bon plan. On conseille alors de reculer, sans toutefois sortir la tige entièrement.

Environ 50% des cellules graisseuses doivent se désagréger in-situ, et ce, progressivement. La fonte est progressive, sur quatre à six semaines et la peau se rétracte à ce rythme. Aucun risque a priori d'excédent cutané résiduel comme dans une liposuccion où la graisse retirée laisse une peau vide qui risque de ne pas se rétracter.

L'autre partie de la graisse est repositionnée à son emplacement initial ; la graisse est aplanie par la spatule 21. La surface est lisse.

Grâce à la canule 7, l'acte se fait sans aucun contact avec l'extérieur (pas de dépose, puis reprise en main par le praticien), évitant ainsi toute contamination, risque d'infection.

D'autres gestes peuvent être associés :
- comblements à l'acide hyaluronique (ou autre produit de comblement) : si le repositionnement du tissu graisseux est insuffisant pour restaurer les volumes perdus.
- remaillages aux fils : lors de l'examen clinique, s'il y a un doute sur les capacités de rétraction de la peau.
- lasers raffermissants.

### 3. Les comblements:

La technique médicale ci-avant permet aussi de pratiquer des actes plus aisés à réaliser, comme les comblements des rides ou l'augmentation des lèvres. Sa forme et sa facilité de manipulation la rendent accessible à des praticiens débutants, sans suites et sans risque d'irrégularités.

Les zones de comblement concernées (par apport de produit de comblement) sont typiquement : les sillons nasogéniens 65, les rides des lèvres 59, les plis de l'amertume 63, les rides du front 73, les rides du lion 71, les pattes d'oie 75, les rides des paupières inférieures 77, les rides jugales 79, les rides du menton 81.

Tracés: repérage des zones et détermination de la quantité de produit nécessaire.

Les injections du produit de comblement se font dans le derme, en rétro-traçant.

Les travées peuvent ne pas être réalisées dans le cas de comblement, dès lors que les injections se font de préférence dans un plan plus superficiel que dans les cas précédents.

De ce qui précède, on aura compris qu'est donc ici concerné un procédé d'intervention dans le derme 3 d'un patient, le procédé comprenant des étapes où :
- on perce la peau par l'intermédiaire d'un mandrin 1 pointu, pour ménager un conduit 5 d'accès vers une zone sous-cutanée 6,
- on introduit, par ce conduit, une canule 7 pourvue d'un tube 13 d'injection ou de prélèvement prolongé par une spatule terminale 21,
- et :
   - par des mouvements, de va-et-vient et/ou en éventail, (d'une tige 13) de la canule, on sépare de leurs attaches les cellules graisseuses (couche 3c) que l'on déplace et répartit par la spatule,
   - ou bien on crée des travées 41 dans le derme, en manipulant la canule suivant des mouvements de va-et-vient et/ou et en éventail, et on y injecte un produit de comblement qu'on lisse par la spatule, en reculant la canule.

En outre, de préférence :
- on injectera le produit de comblement sous le niveau de la spatule, alors que celle-ci est sensiblement à plat (figure 5), et/ou,
- pour percer la peau, on utilisera un mandrin réalisé avec une tige 9 et une poignée 11 par laquelle on tiendra le mandrin, et on percera la peau sur une profondeur d'environ 1,3 cm à 1,8 cm, puis on placera le mandrin dans un logement (25a) de la canule, évitant ainsi de contaminer sa tige.

Et avec le(s) marquage(s) 37 on pourra orienter vers le haut ce(s) marquage(s) en tournant la poignée 33 de la canule, plaçant ainsi le niveau de la spatule au-dessus de celui de l'orifice distal 17b.

Par ailleurs, une fois la/toutes les voies d'accès 5 réalisées via le mandrin 1, on pourra le poser sur un support, avec sa tige 13 dressée à la verticale, libérant ainsi l'accès au tube 13 et donc permettant au tube 23 d'apport du produit de comblement d'être connecté à l'embase creuse 25.

## Revendications

1. Instrument d'intervention sous-cutanée comprenant :
- un mandrin (1) présentant un axe longitudinal suivant lequel s'étend une tige (9) pourvue d'une extrémité d'accès corporel ; et,
- une canule (7) pourvue d'un tube (13) d'injection ou de prélèvement s'étendant suivant un axe longitudinal et terminé, vers des extrémités respectivement proximale et distale, par un orifice (17a, 17b) communiquant avec l'extérieur, et comprenant une spatule (21) terminale qui prolonge le tube (13) à son extrémité distale;
caractérisé en que la canule comprend une embase (25) de raccordement creuse à l'extrémité proximale du tube (13) pour recevoir le mandrin (1) de façon amovible, et en ce que ladite spatule (21) terminale présente une extrémité atraumatique (21a)

2. Instrument selon la revendication 1, où la spatule (21) est plate.

3. Instrument selon la revendication 1 ou 2, où la spatule s'étend dans le prolongement axial du tube (13).

4. Instrument selon l'une des revendications 1 à 3, où le tube (13) présente un diamètre extérieur maximal et, transversalement à l'axe longitudinal, la spatule (21) présente une largeur comprise entre 0,6 et 1,3 fois le diamètre extérieur maximal.

5. Instrument selon l'une des revendications 1 à 3, où le tube (13) présente un diamètre extérieur constant, et, transversalement à l'axe longitudinal, la spatule (21) présente une largeur égale audit diamètre extérieur.

6. Instrument selon l'une des revendications 1 à 5, où, près de l'extrémité distale du tube (13) d'injection ou de prélèvement, l'orifice (17b) du tube (13) est situé dans la paroi latérale dudit tube (13).

7. Instrument selon l'une des revendications 1 à 6, où l'embase (25) de raccordement proximal creuse présente une connexion du type « Luer lock » et à laquelle le tube (13) se raccorde à l'extrémité proximale.

8. Instrument selon l'une des revendications 1 à 7, où l'embase (25) de raccordement proximal creuse de la canule (7) présente une longueur supérieure à la longueur de ladite tige (9), suivant les axes longitudinaux respectifs.

9. Instrument selon l'une des revendications 1 à 7, où la tige (9):
- est effilée à ou en direction de l'extrémité d'accès corporel, suivant ledit axe longitudinal, et/ou
- présente une partie tubulaire rectiligne terminée d'un côté par l'extrémité d'accès corporel qui est conique suivant ledit axe longitudinal.

10. Instrument selon la revendication 7, 8 ou 9, où l'embase (25) de raccordement proximal creuse se termine par une partie évasée vers l'extérieur qui s'étend coaxialement au tube (13) et reçoit le mandrin (1).

11. Instrument selon l'une des revendications 1 à 10, où le mandrin (1) présente, pour ménager ledit conduit (5) d'accès, un diamètre extérieur maximal prédéterminé, et le tube (13) présente un diamètre extérieur maximal supérieur à 1,3 fois celui du mandrin (1).

12. Instrument selon l'une des revendications 1 à 11, où, à l'opposé de l'extrémité d'accès corporel, le mandrin est fixé à une poignée (11) de prise en main terminée, à l'opposé du mandrin (1), par une surface plane d'appui permettant de poser la poignée de façon stable avec le mandrin (1) dressé.

13. Instrument selon l'une des revendications 7 à 11, où :
- l'embase (25) creuse de la canule (7) définit extérieurement une première poignée (33) de prise en main,
- à l'opposé de l'extrémité d'accès corporel, le mandrin (1) est fixé à une seconde poignée (11) de prise en main, et
. les première (33) et seconde (11) poignées sont chacune à facettes, et/ou
. les première (33) et seconde (11) poignées présentent respectivement des premiers et seconds moyens de détrompage angulaire qui, engagés ensemble, bloquent angulairement et de façon réversible les première et seconde poignées l'une par rapport à l'autre et permettent de savoir dans quelle position angulaire est la spatule (21), et
. la seconde poignée (11) et/ou l'embase (25) creuse est pourvue d'un marqueur angulaire, sur une zone visible de dessus quand le tube d'injection ou de prélèvement est angulairement orienté pour que son orifice distal s'ouvre à un niveau inférieur à celui de la spatule (21).

14. Instrument selon l'une des revendications 1 à 13, où le mandrin (1) comprend, à l'opposé de l'extrémité d'accès corporel, une poignée (11) de prise en main ayant une plus grande section que celle de la tige (9).

## Patentansprüche

1. Instrument für subkutane Eingriffe, welches aufweist:
- einen Mandrin (1), der eine Längsachse aufweist, entlang der sich ein Schaft (9) erstreckt, der mit einem Ende für den Körperzugang versehen ist; und
- eine Kanüle (7), die mit einem Injektionsoder Entnahmerohr (13) versehen ist, das sich entlang einer Längsachse erstreckt und an einem proximalen und einem distalen Ende jeweils in einer mit der Außenumgebung in Verbindung stehenden Öffnung (17a, 17b) endet, und die ein spatelförmiges Endstück (21) aufweist, welches das Rohr (13) an seinem distalen Ende verlängert,
**dadurch gekennzeichnet, dass** die Kanüle am proximalen Ende des Rohres (13) einen hohlen Anschlussfuß (25) aufweist, um den Mandrin (1) lösbar aufzunehmen, und dadurch, dass das spatelförmige Endstück (21) ein atraumatisches Ende (21a) aufweist.

2. Instrument nach Anspruch 1, wobei der Spatel (21) flach ist.

3. Instrument nach Anspruch 1 oder 2, wobei sich der Spatel in der axialen Verlängerung des Rohres (13) erstreckt.

4. Instrument nach einem der Ansprüche 1 bis 3, wobei das Rohr (13) einen maximalen Außendurchmesser aufweist und der Spatel (21) quer zur Längsachse eine Breite aufweist, die zwischen dem 0,6- und dem 1,3-Fachen des maximalen Außendurchmessers liegt.

5. Instrument nach einem der Ansprüche 1 bis 3, wobei das Rohr (13) einen konstanten Außendurchmesser aufweist und der Spatel (21) quer zur Längsachse eine Breite aufweist, die gleich dem Außendurchmesser ist.

6. Instrument nach einem der Ansprüche 1 bis 5, wobei in der Nähe des distalen Endes des Injektionsoder Entnahmerohres (13) die Öffnung (17b) des Rohres (13) in der Seitenwand des Rohres (13) angeordnet ist.

7. Instrument nach einem der Ansprüche 1 bis 6, wobei der hohle proximale Anschlussfuß (25) einen Anschluss vom Typ "Luer-Lock" aufweist, an welchen das Rohr (13) am proximalen Ende angeschlossen ist.

8. Instrument nach einem der Ansprüche 1 bis 7, wobei der hohle proximale Anschlussfuß (25) der Kanüle (7) eine Länge aufweist, die, entlang der jeweiligen Längsachsen, größer als die Länge des Schaftes (9) ist.

9. Instrument nach einem der Ansprüche 1 bis 7, wobei der Schaft (9):
- am Ende für den Körperzugang oder in Richtung desselben entlang der Längsachse zugespitzt ist, und/oder
- einen geradlinigen rohrförmigen Teil aufweist, der auf einer Seite in dem Ende für den Körperzugang endet, welches entlang der Längsachse konisch ist.

10. Instrument nach Anspruch 7, 8 oder 9, wobei der hohle proximale Anschlussfuß (25) in einem nach außen aufgeweiteten Teil endet, welcher sich koaxial zum Rohr (13) erstreckt und den Mandrin (1) aufnimmt.

11. Instrument nach einem der Ansprüche 1 bis 10, wobei der Mandrin (1), um den Zugangskanal (5) auszubilden, einen vorbestimmten maximalen Außendurchmesser aufweist und das Rohr (13) einen maximalen Außendurchmesser aufweist, der das 1,3-Fache von demjenigen des Mandrins (1) beträgt.

12. Instrument nach einem der Ansprüche 1 bis 11, wobei der Mandrin gegenüber von dem Ende für den Körperzugang an einem Handgriff (11) befestigt ist, welcher gegenüber von dem Mandrin (1) in einer ebenen Stützfläche endet, die es ermöglicht, den Griff auf stabile Weise mit nach oben gerichtetem Mandrin (1) abzustellen.

13. Instrument nach einem der Ansprüche 7 bis 11, wobei:
- der hohle Fuß (25) der Kanüle (7) außen einen ersten Handgriff (33) definiert,
- der Mandrin (1) gegenüber dem Ende für den Körperzugang an einem zweiten Handgriff (11) befestigt ist, und
• der erste (33) und der zweite (11) Griff jeweils Facetten aufweisen, und/oder
• der erste (33) und der zweite (11) Griff jeweils erste bzw. zweite Winkel-Unverwechselbarkeitsmittel aufweisen, welche, wenn sie sich miteinander in Eingriff befinden, den ersten und den zweiten Griff winkelmäßig und auf eine reversible Weise relativ zueinander blockieren und ermöglichen festzustellen, in welcher Winkelposition sich der Spatel (21) befindet, und
• der zweite Griff (11) und/oder der hohle Fuß (25) mit einer Winkelmarkierung auf einem Bereich versehen ist, der von oben sichtbar ist, wenn das Injektions- oder Entnahmerohr winkelmäßig so ausgerichtet ist, dass sich seine distale Öffnung auf einer Höhe unterhalb von derjenigen des Spatels (21) öffnet.

14. Instrument nach einem der Ansprüche 1 bis 13, wobei der Mandrin (1) auf der dem Ende für den Körperzugang gegenüberliegenden Seite einen Handgriff (11) mit einem Querschnitt aufweist, der größer als derjenige des Schaftes (9) ist.

## Claims

1. Instrument for subcutaneous intervention, comprising:
- a mandrel (1) having a longitudinal axis along which there extends a rod (9) provided with a bodily-access end; and
- a canula (7) provided with an injection or sampling tube (13) extending along a longitudinal axis and ending, towards respectively a proximal end and a distal end, in an orifice (17a, 17b) that communicates with the outside, and comprising an end spatula (21) which extends the tube (13) at its distal end;
**characterized in that** the canula comprises a hollow connection base (25) for connection to the proximal end of the tube (13) in order to accept the mandrel (1) removably, and **in that** the said end spatula (21) has an atraumatic end (21a).

2. Instrument according to Claim 1, in which the spatula (21) is flat.

3. Instrument according to Claim 1 or 2, in which the spatula extends in the axial continuation of the tube (13).

4. Instrument according to one of Claims 1 to 3, in which the tube (13) has a maximum outside diameter and, transversely to the longitudinal axis, the spatula (21) has a width of between 0.6 and 1.3 times the maximum outside diameter.

5. Instrument according to one of Claims 1 to 3, in which the tube (13) has a constant outside diameter and, transversely to the longitudinal axis, the spatula (21) has a width equal to the said outside diameter.

6. Instrument according to one of Claims 1 to 5, in which, near the distal end of the injection or sampling tube (13), the orifice (17b) of the tube (13) is situated in the lateral wall of the said tube (13).

7. Instrument according to one of Claims 1 to 6, in which the hollow proximal connection base (25) has a connection of the "Luer lock" type and to which the tube (13) connects at the proximal end.

8. Instrument according to one of Claims 1 to 7, in which the hollow proximal connection base (25) of the canula (7) has a length greater than the length of the said rod (9), measured along the respective longitudinal axes.

9. Instrument according to one of Claims 1 to 7, in which the rod (9):
- is tapered at or towards the bodily-access end, along the said longitudinal axis, and/or
- has a rectilinear tubular part ending at one end in the bodily-access end which is conical along the said longitudinal axis.

10. Instrument according to Claim 7, 8 or 9, in which the hollow proximal connection base (25) ends in an outwardly flared part which extends coaxial to the tube (13) and accepts the mandrel (1).

11. Instrument according to one of Claims 1 to 10, in which the mandrel (1) has, in order to form the said access conduit (5), a predetermined maximum outside diameter and the tube (13) has a maximum outside diameter greater than 1.3 times that of the mandrel (1).

12. Instrument according to one of Claims 1 to 11, in which, at the opposite end to the bodily-access end, the mandrel is fixed to a hand grip (11) ending, at the opposite end to the mandrel (1), in a planar bearing surface that allows the hand grip to be put down in a stable manner with the mandrel (1) standing up straight.

13. Instrument according to one of Claims 7 to 11, in which:
- the hollow base (25) of the canula (7) externally defines a first hand grip (33),
- at the opposite end to the bodily-access end, the mandrel (1) is fixed to a second hand grip (11), and
• the first hand grip (33) and second hand grip (11) are each faceted, and/or
• the first hand grip (33) and the second hand grip (11) respectively have first and second angular poke yoke means which, when fitted together, angularly and reversibly block the first and second hand grips the one relative to the other and make it possible to determine what angular position the spatula (21) is occupying, and
• the second hand grip (11) and/or the hollow base (25) is provided with an angular marker on a zone visible from the top when the injection or sampling tube is angularly oriented so that its distal orifice opens at a level lower than that of the spatula (21).

14. Instrument according to one of Claims 1 to 13, in which the mandrel (1) comprises, at the opposite end to the bodily-access end, a hand grip (11) that has a cross section larger than that of the rod (9).
